# EUROPEAN PATENT APPLICATION

(11) **EP 4 708 308 A1**
(43) Date of publication of application: **11.03.2026**
(21) Application number: 24382970.2
(22) Date of filing: 10.09.2024
(51) Int. Cl.: G16H 20/10, G16H 50/70

(54) **SYSTEMS METHODS FOR BIOMARKER ESTIMATION**

(71) Applicant: Roche Diabetes Care GmbH, 68305 Mannheim (DE)
(72) Inventor: ANDORRA INGLES, Magi, 08174 Sant Cugat del Valles, Barcelona (ES); HERRERO VINAS, Paul, 08174 Sant Cugat del Valles, Barcelona (ES)
(74) Representative: Altmann Stößel Dick Patentanwälte PartG mbB

(57) **Abstract**

A method for biomarker prediction has been developed. The method includes providing a set of program instructions which receive data corresponding to at least one measurement of a biomarker value in a body of a user, generate a first prediction of a future value of the biomarker in the body of the user based on the at least one measurement applied to a physics-based model, generate an estimated error of the first prediction based on the at least one measurement using a trained machine learning model, correct the first prediction based on the estimated error to generate a second prediction of the future value of the biomarker in the body of the user, and generate an output indicating the second prediction of the future value of the biomarker in the body of the user.

## Description

### TECHNICAL FIELD

This disclosure is directed to improved systems and methods for the predictions of biomarkers in patients using physics-based and machine learning models.

### BACKGROUND

The state of the art in medical diagnostic and prognostics utilizes physics-based models to provide systematic predictions for the physiology of a patient based on known biophysical properties of human physiology and the measurement of one or more biomarkers in the patient. Physics-based models enable the prediction of various biological processes in the human body and are utilized for pharmaceutical development to simulate the pharmacokinetic and pharmacodynamic effects of a medicament in a patient's body. For example, physics-based models can generate a prediction for the future blood glucose level of a person with diabetes (PwD) over various time ranges using systems of differential equations or other mathematical equations that describe the physical process for metabolism of glucose. The physics-based model receives input biomarker data to characterize the PwD's current and past blood glucose levels and optionally other pertinent biomarkers such as administration of insulin or other medications and the consumption of carbohydrates to generate the prediction. In modern applications, computers typically perform the computations of the physics-based model and produce an output that may be used by healthcare providers or directly by the patient, such as a PwD in the example of blood glucose prediction.

While physics-based models that are known to the art provide useful medical results, another form of predictive model that has gained recent prominence utilizes machine learning (ML) models, including neural networks, to provide similar predictions to the physics-based models. Unlike the physics-based models, which are rooted in known physical equations that describe the biophysical mechanisms of the human body, the ML models generally provide a structure that encodes information received from a training data set. These ML models undergo a training process that uses a large amount of empirical data to train the ML models to produce accurate predictive results. If properly trained, the ML models encode latent information in an implicit manner that generally corresponds to the same physical processes that the physics-based models describe explicitly.

While both physics-based models and ML models can be useful in a medical context, both model types also have drawbacks. The physic-based models may lack precision when certain biophysical processes in the body are not expressly incorporated into the physics-based model. In some circumstances, the ML models may model some biophysical processes in the body that are missed in the physical models, but because the ML models are inherently statistical, in some circumstances these models produce inaccurate outputs when the ML model receives input data that are not properly incorporated into the earlier training process. Previous combinations of physics-based models and ML models focus on using a trained ML model to generate a prediction while a physics-based model generates another prediction that serves to check the plausibility of the output from the ML model to avoid occasional results with very large errors. However, further improvements to systems and methods for utilizing both physics-based models and ML models in biomarker prediction would be beneficial.

### SUMMARY

In one embodiment, a method for biomarker prediction has been developed. The method includes providing a set of program instructions which, when stored in a non-transitory memory and executed by a processor in a computing system, configure the computing system to, receive data corresponding to at least one measurement of a biomarker value in a body of a user, generate a first prediction of a future value of the biomarker in the body of the user based on the at least one measurement applied to a physics-based model, generate an estimated error of the first prediction based on the at least one measurement using a trained machine learning model, correct the first prediction based on the estimated error to generate a second prediction of the future value of the biomarker in the body of the user, and generate an output indicating the second prediction of the future value of the biomarker in the body of the user.

In another embodiment a method for training a machine learning model for use in predicting biomarker value has been developed. The method includes receiving, with a training system, a training data set, the training data set further including a plurality of biomarker measurement sequences, generating, with the training system, at least one predicted biomarker value for each biomarker measurement sequence based on a first portion of each biomarker measurement sequence using a physics-based model, identifying, with the training system, a ground-truth error in the at least one predicted biomarker value for each biomarker measurement sequence based on a second portion of each biomarker measurement sequence, and performing, with the training system, one or more training operations in an iterative manner to generate a trained machine learning model from the untrained machine learning model. In the method, each training operation includes applying, with the training system, the first portion of each biomarker measurement sequence to an untrained machine learning model to generate a plurality of predicted error values corresponding to each biomarker measurement sequence, and adjusting a plurality of parameter values in the untrained machine learning model based on a difference between the predicted error value and the ground-truth error value corresponding to each biomarker measurement sequence to optimize the output of the untrained machine learning model.

One or both of the methods may be computer implemented. The term "computer implemented" as used herein is a broad term and is to be given its ordinary and customary meaning to a person of ordinary skill in the art and is not to be limited to a special or customized meaning. The term specifically may refer, without limitation, to a method involving at least one computer and/or at least one computer network. The computer and/or computer network may comprise at least one processor which is configured for performing at least one of the method steps of one or both of the methods according to the present invention. Preferably each of the method steps is performed by the computer and/or computer network. One or both of the methods may be performed completely automatically, specifically without user interaction.

Further disclosed and proposed herein is a computer program including computer-executable instructions for performing one or both of the method according to the present invention in one or more of the embodiments enclosed herein when the instructions are executed on a computer or computer network. Specifically, the computer program may be stored on a computer-readable data carrier and/or on a computer-readable storage medium.

As used herein, the terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

Thus, specifically, one, more than one or even all of method steps as indicated above may be performed by using a computer or a computer network, preferably by using a computer program.

Further disclosed and proposed herein is a computer program product having program code means, in order to perform one or both of the methods according to the present invention in one or more of the embodiments enclosed herein when the program is executed on a computer or computer network. Specifically, the program code means may be stored on a computer-readable data carrier and/or on a computer-readable storage medium.

Further disclosed and proposed herein is a data carrier having a data structure stored thereon, which, after loading into a computer or computer network, such as into a working memory or main memory of the computer or computer network, may execute one or both of the methods according to one or more of the embodiments disclosed herein.

Further disclosed and proposed herein is a non-transient computer-readable medium including instructions that, when executed by one or more processors, cause the one or more processors to perform one or both of the methods according to one or more of the embodiments disclosed herein.

Further disclosed and proposed herein is a computer program product with program code means stored on a machine-readable carrier, in order to perform one or both of the methods according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier and/or on a computer-readable storage medium. Specifically, the computer program product may be distributed over a data network.

Finally, disclosed and proposed herein is a modulated data signal which contains instructions readable by a computer system or computer network, for performing one or both of the methods according to one or more of the embodiments disclosed herein.

Referring to the computer-implemented aspects of the invention, one or more of the method steps or even all of the method steps of one or both of the methods according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Specifically, further disclosed herein are:
- a computer or computer network comprising at least one processor, wherein the processor is adapted to perform one or both of the methods according to one of the embodiments described in this description,
- a computer loadable data structure that is adapted to perform one or both of the methods according to one of the embodiments described in this description while the data structure is being executed on a computer,
- a computer program, wherein the computer program is adapted to perform t one or both of the methods according to one of the embodiments described in this description while the program is being executed on a computer,
- a computer program comprising program means for performing one or both of the methods according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform one or both of the methods according to one of the embodiments described in this description after having been loaded into a main and/or working storage of a computer or of a computer network, and
- a computer program product having program code means, wherein the program code means can be stored or are stored on a storage medium, for performing one or both of the methods according to one of the embodiments described in this description, if the program code means are executed on a computer or on a computer network.

Summarizing and without excluding further possible embodiments, the following embodiments may be envisaged:
Embodiment 1. A method for biomarker prediction comprising:
   providing a set of program instructions which, when stored in a non-transitory memory and executed by a processor in a computing system, configure the computing system to:
   receive data corresponding to at least one measurement of a biomarker value in a body of a user;
   generate a first prediction of a future value of the biomarker in the body of the user based on the at least one measurement applied to a physics-based model;
   generate an estimated error of the first prediction based on the at least one measurement using a trained machine learning model;
   correct the first prediction based on the estimated error to generate a second prediction of the future value of the biomarker in the body of the user; and
   generate an output indicating the second prediction of the future value of the biomarker in the body of the user.
Embodiment 2. The method of embodiment 1, wherein the biomarker is glucose.
Embodiment 3. The method according to any one of the preceding embodiments, wherein the physics-based model is a Patek model.
Embodiment 4. The method according to any one of the preceding embodiments, wherein the physics-based model is a Patek model that incorporates a Kalman filter.
Embodiment 5. The method according to any one of the preceding embodiments, wherein the physics-based model is a Bergman model.
Embodiment 6. The method according to any one of the preceding embodiments, wherein the at least one measurement of the biomarker value in the body of the user further comprises a time series of biomarker measurements received from a continuous biomarker measurement device coupled to the body of the user.
Embodiment 7. The method according to any one of the preceding embodiments, wherein the trained machine learning model is a recurrent neural network (RNN).
Embodiment 8. The method of embodiment 7, wherein the RNN further comprises an encoder, and a decoder.
Embodiment 9. The method according to any one of the preceding embodiments, wherein the program instructions further configure the computing system to:
   generate the estimated error of the first prediction as a probability distribution corresponding to an output of the trained machine learning model; and
   correct the first prediction based on a plurality of estimated errors having probability values exceeding a predetermined threshold in the probability distribution to generate the second prediction of the future value of the biomarker as a range of values.
Embodiment 10. A method for training a machine learning model for use in predicting biomarker value comprising:
   receiving, with a training system, a training data set, the training data set further comprising a plurality of biomarker measurement sequences;
   generating, with the training system, at least one predicted biomarker value for each biomarker measurement sequence based on a first portion of each biomarker measurement sequence using a physics-based model;
   identifying, with the training system, a ground-truth error in the at least one predicted biomarker value for each biomarker measurement sequence based on a second portion of each biomarker measurement sequence; and
   performing, with the training system, one or more training operations in an iterative manner to generate a trained machine learning model from the untrained machine learning model, each training operation comprising:
      applying, with the training system, the first portion of each biomarker measurement sequence to an untrained machine learning model to generate a plurality of predicted error values corresponding to each biomarker measurement sequence; and
      adjusting a plurality of parameter values in the untrained machine learning model based on a difference between the predicted error value and the ground-truth error value corresponding to each biomarker measurement sequence to optimize the output of the untrained machine learning model.
Embodiment 11. The method of embodiment 10, wherein the training system performs a gradient descent optimization to adjust the plurality of parameter values in the untrained machine learning model during each training operation.
Embodiment 12. The method according to any one of the preceding embodiments relating to a method for training a machine learning model further comprising:
   performing the one or more training operations a predetermined number of times to generate the trained machine learning model.
Embodiment 13. The method according to any one of the preceding embodiments relating to a method for training a machine learning model further comprising:
   performing the one or more training operations iteratively until the plurality of parameter values converge over at least two successive training operations.
Embodiment 14. The method according to any one of the preceding embodiments relating to a method for training a machine learning model further comprising:
   performing the one or more training operations iteratively until the plurality of predicted error values corresponding to each biomarker measurement sequence converge over at least two successive training operations.
Embodiment 15. The method according to any one of the preceding embodiments relating to a method for training a machine learning model, wherein the biomarker is glucose.
Embodiment 16. The method according to any one of the preceding embodiments relating to a method for training a machine learning model, wherein the training data set further comprises a plurality of time series of biomarker measurements received from a plurality of continuous biomarker measurement devices, each continuous biomarker measurement device being coupled to a body of a training user in a plurality of training users.
Embodiment 17. The method according to any one of the preceding embodiments relating to a method for training a machine learning model, wherein the untrained machine learning model is an untrained recurrent neural network (RNN) and the trained machine learning model is a trained RNN.
Embodiment 18. The method according to any one of the preceding embodiments relating to a method for training a machine learning model, wherein the physics-based model is a Patek model.
Embodiment 19. The method according to any one of the preceding embodiments relating to a method for training a machine learning model, wherein the physics-based model is a Patek model that incorporates a Kalman filter.
Embodiment 20. The method according to any one of the preceding embodiments relating to a method for training a machine learning model, wherein the physics-based model is a Bergman model.
Embodiment 21. A system for biomarker prediction comprising:
   a memory configured to store program instructions, a physics-based model, a trained machine learning model, and data corresponding to at least one measurement of a biomarker value in a body of a user;
   an output device; and
   a processor operatively coupled to the memory and the output device, the processor being configured to:
      execute the stored program instructions to perform any of the methods of embodiments 1 - 9 to generate an output of the second prediction of the future value of the biomarker in the body of the user using the physics-based model and the trained machine learning model with the output device.
Embodiment 22. A system for training a machine learning model for use in predicting biomarker value comprising:
   a memory configured to store program instructions, a physics-based model, an untrained machine learning model, and a training data set; and
   a processor operatively coupled to the memory, the processor being further configured to:
      execute the stored program instructions to perform any of the methods of embodiments 10 - 20 to generate a trained machine learning model using the physics-based model, the untrained machine learning model, and the training data set.
Embodiment 23. A computer program comprising instructions which, when the program is executed by the system for biomarker prediction according to embodiment 21, cause the system for biomarker prediction to perform the method according to any one of embodiments 1 - 9.
Embodiment 24. A computer program comprising instructions which, when the program is executed by the system for training a machine learning model according to embodiment 22, cause the system for training a machine learning model to perform the method according to any one of embodiments 10 - 20.
Embodiment 25. A computer-readable storage medium comprising instructions which, when the instructions are executed by the system for biomarker prediction according to embodiment 21, cause the system for biomarker prediction to perform the method according to any one of embodiments 1 - 9.
Embodiment 26. A computer-readable storage medium comprising instructions which, when the instructions are executed by the system for training a machine learning model according to embodiment 22, cause the system for training a machine learning model to perform the method according to any one of embodiments 10-20.
Embodiment 27. 23.A non-transient computer-readable medium including instructions that, when executed by one or more processors, cause the one or more processors to perform one or both of the methods according to any one of the preceding embodiments referring to a method.

### BRIEF DESCRIPTION OF THE SEVERAL VIEWS OF THE DRAWINGS

To easily identify the discussion of any particular element or act, the most significant digit or digits in a reference number refer to the figure number in which that element is first introduced.
FIG. 1 is a schematic diagram of a system for generating one or more predictions of a biomarker using a physics-based model to predict the biomarker and a machine learning (ML) model that generates estimates of residual errors in the prediction from the physics-based model.
FIG. 2A is a block diagram of a method for training the ML model in the system of FIG. 1.
FIG. 2B is a diagram of a process for generating one or more predictions of a biomarker using the physics-based model and trained ML model in the system of FIG. 1.
FIG. 3 is a diagram of an exemplary ML model used in the system of FIG. 1.
FIG. 4 is a graph depicting root mean square error results for biomarker predictions generated using the system of FIG. 1 compared to errors generated by a physics-based model and to a machine-learning model.

### DETAILED DESCRIPTION

These and other advantages, effects, features and objects are better understood from the following description. In the description, reference is made to the accompanying drawings, which form a part hereof and in which there is shown by way of illustration, not limitation, embodiments of the inventive concept. Corresponding reference numbers indicate corresponding parts throughout the several views of the drawings.

While the inventive concept is susceptible to various modifications and alternative forms, exemplary embodiments thereof are shown by way of example in the drawings and are herein described in detail. It should be understood, however, that the description of exemplary embodiments that follows is not intended to limit the inventive concept to the particular forms disclosed, but on the contrary, the intention is to cover all advantages, effects, and features falling within the spirit and scope thereof as defined by the embodiments described herein and the embodiments below. Reference should therefore be made to the embodiments described herein and embodiments below for interpreting the scope of the inventive concept. As such, it should be noted that the embodiments described herein may have advantages, effects, and features useful in solving other problems.

The devices, systems and methods now will be described more fully hereinafter with reference to the accompanying drawings, in which some, but not all embodiments of the inventive concept are shown. Indeed, the devices, systems and methods may be embodied in many different forms and should not be construed as limited to the embodiments set forth herein; rather, these embodiments are provided so that this disclosure will satisfy applicable legal requirements.

Likewise, many modifications and other embodiments of the devices, systems and methods described herein will come to mind to one of skill in the art to which the disclosure pertains having the benefit of the teachings presented in the foregoing descriptions and the associated drawings. Therefore, it is to be understood that the devices, systems and methods are not to be limited to the specific embodiments disclosed and that modifications and other embodiments are intended to be included within the scope of the embodiments. Although specific terms are employed herein, they are used in a generic and descriptive sense only and not for purposes of limitation.

Unless defined otherwise, all technical and scientific terms used herein have the same meaning as commonly understood by one of skill in the art to which the disclosure pertains. Although any methods and materials similar to or equivalent to those described herein can be used in the practice or testing of the methods, the preferred methods and materials are described herein.

Moreover, reference to an element by the indefinite article "a" or "an" does not exclude the possibility that more than one element is present, unless the context clearly requires that there be one and only one element. The indefinite article "a" or "an" thus usually means "at least one." Likewise, the terms "have," "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. For example, the expressions "A has B," "A comprises B" and "A includes B" may refer both to a situation in which, besides B, no other element is present in A (*i*.*e*., a situation in which A solely and exclusively consists of B) or to a situation in which, besides B, one or more further elements are present in A, such as element C, elements C and D, or even further elements.

As used herein, the term "Person with Diabetes" (PwD) refers to a patient who is diagnosed with or is at-risk for being diagnosed with one or more forms of diabetes including pre-diabetes, type 1 diabetes, type 2 diabetes, gestational diabetes, and, optionally, one or more comorbidities that are associated with diabetes.

As used herein, the term "physics-based model" applies to any model that uses one or more linear equations, non-linear equations, differential equations, stochastic processes, or combinations thereof to provide predictions of current or future biomarker values in a human body where the equations in the physics based correspond to observed biophysical processes in humans or animals. In some non-limiting examples, physics-based models includes one or more systems of equations that model one or more metabolic processes such as glucose-insulin regulation and metabolic absorption of a meal, or absorption of a medicament such as subcutaneous insulin absorption, where the equations are based on empirical observations of these biophysical processes. In addition to physics-based models that directly model human or animal physiology, in the context of this application the term physics-based model also applies to other predictive models that rely on systems of one or more linear equations, non-linear equations, differential equations, stochastic processes, or combinations thereof to provide predictions of current or future biomarker values based on one or more previously identified biomarker values but not necessarily directly modeling biophysical processes in a human or other animal. For example, auto-regressive models that include a stochastic term to generate predictions based on prior inputs in the presence of a stochastic noise source are also considered physics-based models when adapted to predicting biomarker values even if they do not directly model biophysical processes.

As used herein, the term "biomarker" refers to any quantifiable aspect of a subject's physiology or medical treatment that is either identified directly or calculated from one or more measurements generated by an analytical device or received from another input source. In the embodiments described herein, measured biomarker data is provided as input to physics-based and machine learning models, which generate one or more predictions of current or future biomarker information beyond the values of the measured biomarker input data. For example, the glucose levels in the body of a PwD are one example of a biomarker that are measured using an analytical device, such a spot or continuous glucose monitor, and the physics-based and machine learning models generate predictions of future blood glucose biomarker levels. Other biomarkers may include information pertaining to insulin or other medication dosages administered to the PwD, exercise or other physical activity that the PwD performs, carbohydrates that the PwD consumes, or any other data pertinent to predicting changes in the blood glucose levels of the PwD over time. For a PwD, a biomarker of interest may be directly linked to monitoring one or more of diabetes in the PwD, a diabetes-related comorbidity in the PwD, or to the general health and wellness of the PwD. While the specific embodiments described herein use biomarkers associated with PwDs as examples, those of skill in the art will note that the teachings herein are applicable physics-based models and corresponding machine learning residual error estimation models that predict the values of other biomarkers as well.

As used herein, the terms "residual", "error", and "residual error" are used interchangeably unless otherwise noted and refer to the directly measured or predicted difference between a biomarker prediction generated by a physics-based model and the actual value of the biomarker. As described in further detail below, during a training process for a machine learning model the training data include directly measured "ground-truth" error data that enable the direct measurement of the residual error between outputs of the physics-based model and actual biomarker levels, which are used to train the ML model to predict the residual errors during later inferencing operations.

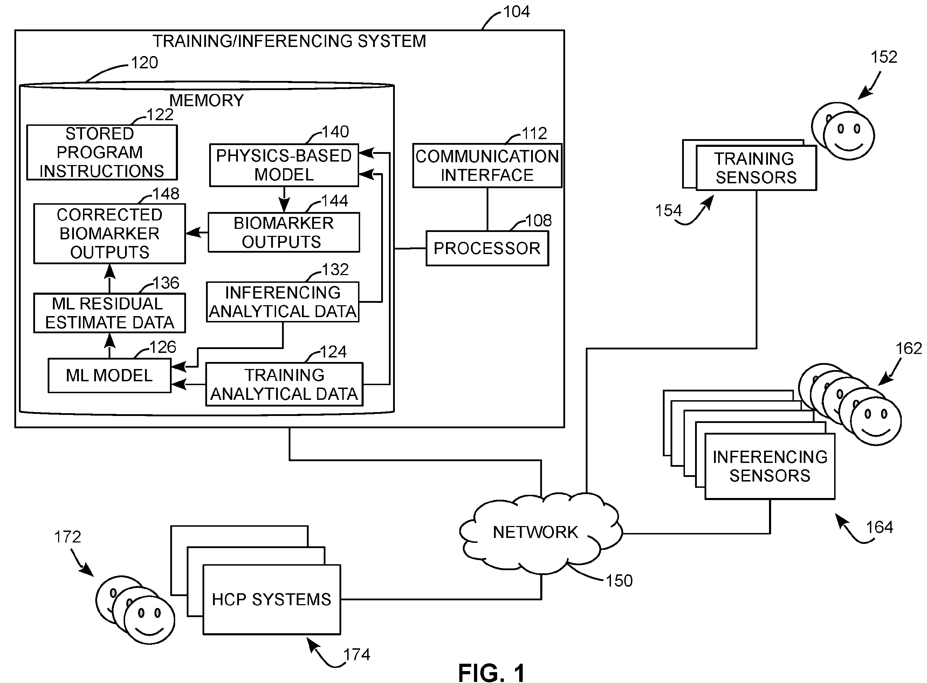

Fig. 1 depicts a system 104 for generating one or more predictions of a biomarker using a physics-based model to predict the biomarker and a machine learning (ML) model that generates estimates of residual errors in the prediction from the physics-based model. Fig. 1 further depicts a plurality of training users 152 with corresponding training sensors 154, a plurality of inferencing users 162 with corresponding inferencing sensor device systems 164, and a plurality of healthcare providers (HCPs) 172 with corresponding HCP computing terminals 174.

In FIG. 1, the training sensors 154 collect training sensor data from the training users 152 during a training process for a machine learning model. The inferencing sensor device systems 164 collect sensor data from the inferencing users 162 after the machine learning model is trained, and the trained machine learning model produces an output indicative of a biomarker in the data. In some embodiments, the hardware configuration of the training sensors 154 and the inferencing sensor device systems 164 is identical. In other embodiments, the inferencing sensor device systems 164 either incorporate or are communicatively coupled to additional hardware elements to perform inferencing operations. As such, references to the inferencing sensor device systems 164 herein refer to both the sensor hardware and additional hardware and software elements that interface with the sensor including, for example, a spot monitoring blood glucose meter ("BGM"), a continuous glucose monitor ("CGM"), or other diagnostic biomarker measurement device used by the inferencing users 162. In most practical embodiments the inferencing users 162 represent a larger population that the training users 152, although it is envisioned that some or all of the training users 152 may also be inferencing users 162 upon completion of the training process.

In the configuration of FIG. 1, the training system 104 includes a processor 108, communication interface 112, and a memory 120. The processor 108 is formed from one or more digital processing devices such as central processing units (CPUs), graphics processing units (GPUs), field programmable gate arrays (FPGAs), application specific integrated circuits (ASICs), digital signal processors (DSPs) and any other suitable digital logic devices. In some embodiments, the processor 108 optionally includes one or more neural network accelerators, which are also referred to as neural processing units (NPUs), that are configured to perform either or both of machine learning model training and machine learning model inferencing operations in an efficient manner, although these accelerators are not strictly required to implement the system 104 and to perform the methods described herein. The communication interface 112 is a device that enables the training system 104 to communicate with other computing systems, including the training sensors 154, inferencing sensor device systems 164, and the HCP terminals 174, via the network 150. Non-limiting examples of the communication interface 112 include wired and wireless network adapters such as Ethernet and IEEE 802.11 family wireless network adapters. The network 150 is typically a local area network (LAN) or wide area network (WAN) such as the Internet. While the network 150 enables an expedient remote exchange of data between the training/inferencing system 104 and the sensors 154/164, in an alternative configuration the network 150 is a physical "sneakernet" that enables the exchange of data storage devices such as USB memory sticks or other data storage media to enable the training/inferencing system 104 to receive blood glucose data from the sensors 154/164 by directly accessing the data storage media.

In the configuration of FIG. 1, both the training sensors 154 and the inferencing sensor systems 164 include at least one sensor that is configured to measure a biomarker when coupled to a corresponding one of the training users 152 or inferencing users 162. In some embodiments the training sensors 154 and the inferencing sensors systems include a continuous biomarker measurement device, which generates a series of biomarker measurements over time. Examples of continuous biomarker measurement devices include, but are not limited to, continuous glucose monitors, pulse-oximeter device, cardiac monitors, fitness monitors, and the like. In other embodiments, the training sensors 154 and the inferencing sensor systems 164 include a spot monitoring device, such as a blood glucose meter, which generates a single biomarker measurement when a corresponding training user 152 or inferencing user 162 operates the spot monitoring device.

In the training system 104, the memory 120 is a non-transitory memory system that includes one or more data storage devices including at least one of a non-volatile or volatile memory device configured to store program instructions 122, training analytical data 124, a machine learning (ML) model 126, inferencing analytical data 132, residual error estimate output data 136, a physics-based model 140, primary predicted biomarker output data 144, and corrected biomarker output data 148. The training analytical data 124 optionally include additional ground-truth data about a biomarker of interest for a supervised training process, which enables the training system 104 to verify the progress of training the ML model 126 from an initial untrained state to a trained state for the estimation of errors in the primary biomarker prediction data 144. The ground-truth data are typically generated using independent sensing devices (not shown) that the training users 152 operate concurrently with the operation of the sensors 154, but after completion of the training process the inferencing users 162 operate the inferencing sensor device systems 164 with the trained ML model 126 without the use of additional independent sensing devices. Furthermore, the training analytical data 124 optionally include additional verification data that are not used directly to train the ML model 126, but that are used to verify that the ML model 126 has been properly trained. The stored program instructions 122 include instructions for a training process that trains the ML model 126 to predict residual errors in outputs of the physics-based model 140 based on the observed residual errors in the primary biomarker output data 144 and the training analytical data 124. The stored program instructions 122 optionally include instructions to implement inferencing operations using the physics-based model 140 and the ML model 126 once the ML model 126 is trained.

In the illustrative embodiments described herein, the physics-based model 140 is a model that is a modification of a prior-art model known as a "Bergman minimal model" based on a model developed by Patek et al. (the "Patek model") and described in a publication entitled "Empirical Representation of Blood Glucose Variability in a Compartmental Model" published in "Prediction methods for blood glucose concentration: Design, use and evaluation," pp. 133-157, © 2016. The Patek model incorporates three ordinary differential equation sub-models, one for glucose-insulin regulation, subcutaneous insulin absorption, and a meal absorption model. To individualize the Patek model, three parameters, one of the glucose-insulin model (SI), and two of the glucose absorption model (k_{abs}, kₜₐᵤ), were identified by using a cost function that minimizes the root mean squared error (RMSE) between the glucose prediction time series and the measurements from a continuous glucose monitor (CGM) device generated at five minute increments (e.g. 5, 10,...,115, 120 min). For this purpose, a two-weeks data scenario was employed. In the embodiment of FIG. 1, the physics-based model 140 extends the Patek model to incorporate an optional Kalman filter that estimates the states of the Patek model based on the measurement data and uncertainty in the estimates generated by the model, which can reduce errors in the model output. However, the physics-based model 140 still generates errors that are outside of the correction capabilities of the Kalman filter. Non-limiting alternative examples of physics-based models for blood glucose prediction that are known to the art are the use of other glucose regulatory models, such as, the Bergman minimal model or the Hovorka model, and the use of a non-stochastic model state estimator, such as the Luenberger observer, instead of the Kalman filter. More broadly, the system 104 may be adapted to train the ML model to estimate errors in the predictions for biomarkers from a wide range of physics-based models, and the embodiments described herein pertaining to blood glucose prediction are non-limiting examples.

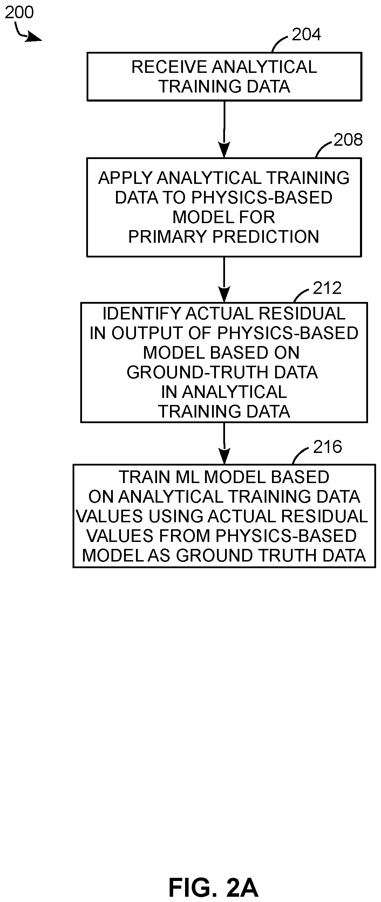

FIG. 2A is a block diagram of a process 200 for training the ML model in the system of FIG. 1. In the description below, a reference to the process 200 performing a function or action refers to the operation of a processor to execute stored program instructions to perform the function or action in coordination with other components of a biomarker prediction and machine learning system. In the illustrative embodiment of FIG. 2A, the process 200 trains an ML model to identify residual errors from a physics-based model that predicts future blood glucose levels in a PwD, but those of skill in the art will recognize the applicability of this process to physics-based prediction models for other biomarkers as well. The process 200 is described in conjunction with the embodiment of FIG. 1 and the system 104 acting as a training system for illustrative purposes.

The process 200 begins as the system 104 receives analytical training data (block 204). The training users 152 operate the training sensors 154 to generate a series of glucose measurements that are transmitted to the system 104 via the network 150. In one configuration, the training sensors are CGMs that are configured to generate glucose measurements at regular intervals (e.g. once every 5 minutes), in another configuration the training sensors 154 are spot BGMs that the training users 152 operate at regular intervals to perform blood glucose measurements, while in still another configuration the training users 152 use a combination of CGMS and BGMs to generate the analytical training data. In some configurations, the system 104 optionally receives analytical data corresponding to the dosages of insulin or medication that are administered during the collection process, the consumption of carbohydrates during the collection process, and data corresponding to exercise or other activities that the training users perform during the collection process. In some embodiments, the training sensors 154 further include automated data measurement devices, such as medication pumps or "smart" pens, record the administration of insulin or other medications, software logging applications record the consumption of carbohydrates, and fitness tracker devices record physical activity to automate some or all of the analytical data collection process. While different configurations of the process 200 may vary the amount of analytical data collected from the training users 152, the analytical training data set typically includes multiple recordings of each user over a series of several hours including waking and sleeping periods, fasting and prandial periods, and periods of low and high activity levels. Additionally, it is preferable to receive analytical data from the training users 152 that includes episodes of hyperglycemia and hypoglycemia, although these data may be generated synthetically in the event that generating the analytical data corresponding to the hyperglycemic and hypoglycemic events could compromise the health and safety of the test subjects.

During the training process, the system 104 partitions the training data into a sets of "prior" and "future" analytical training data. For example, a four hour series of glucose measurements in the training data may be partitioned into two hours of "prior" data that form the input for the physics-based model 140 to predict an additional two hours of "future" data. The second "future" data set in the training data serves as ground-truth data to determine the residual errors of the predictions from the physics-based model 140, and these residual errors in turn serve as targets to train the ML model 126. Preferably, the collected analytical training data also include validation data sets. The validation data sets have the same general structure as data sets used for training, but they are not used in direct ML model training and are preferably collected from training users 152 who are optionally distinct from the training users 152 whose collected data are used in the training process. Instead, the validation data sets provide additional sets of data with known values that enable verification that the ML model is effectively trained to perform inference operations on data that are not directly used to train the ML model.

In one specific training configuration, a glucose data corresponding to a group of 25 subjects from an existing data source, such as the commercially available Tidepool platform, were selected based on data quality. In particular, CGM data loss was required to be less than 5%, the average number of meals per day to be more than three (3), and the average number of insulin boli per day to be more than four (4). While the training data set described in this example includes only insulin-dependent PwDs, other training data sets may be generated from a mixture of insulin-dependent PwDs and non-insulin-dependent PwDs, or only non-insulin-dependent PwDs based on the intended users.

During the process 200, the system 104 applies the analytical training data to the physics-based model to generate outputs that are used for primary prediction of a biomarker (block 208). In the example of FIG. 1, processor 108 applies the training analytical data 124 to the physics-based model 140, which generates a series of predicted biomarker output data 144, such as a prediction of future blood glucose values based on a past series of blood glucose measurements in the training analytical data 124. The physics-based model 140 is said to produce a "primary" prediction here because this model produces the initial prediction for the biomarker while the ML model 126 produces a prediction of the residual error estimate 136 that is combined with the biomarker output data 144 to form the final corrected biomarker output data 148.

During the process 200, the system 104 also identifies the ground-truth residual data in the output of the physics-based model (block 212). To identify the ground-truth residual data, the processor 108 compares the biomarker output data 144 from the physics-based model 140 to the corresponding future time training analytical data 124, which form the ground-truth data since these data are based on direct measurements from the training sensors 154. For example, in one configuration the physics-based model 140 generates a prediction sequence including a series of blood glucose values for a PwD over a time period (e.g. two hours) based on an input of previously observed blood glucose values and, optionally, other past observed data in the "prior" portion of the training analytical data 124. To identify the ground-truth residual data, the processor 108 compares these predicted biomarkers with the "future" portion of the training analytical data 124 that record the actual observations for the blood glucose values over the same time period. The differences between the predicted biomarker output data 144 and the observed training analytical data 124 form the ground-truth residual error data, which the system 104 then uses to determine the accuracy of predictions from the ML model 126 during training. The system 104 generates the ground-truth residual error data for multiple data sets that cover multiple training users 152 and cover variations in the blood glucose levels of each training user over time to provide a sufficiently large and diverse set of ground-truth residual error values in order to train the ML model 126.

The process 200 continues as the ML model is trained to predict residual values from the physics-based model using actual residual values measured from the physics-based model as training data (block 216). In one configuration, the system 104 trains the ML model 126 to apply the analytic training data to the ML model to generate candidate output values that represent the predicted residual errors from the corresponding predicted biomarker data from the physics-based model 140. The system 104 compares output of the ML model 126 to the ground-truth residual error data and uses a gradient descent optimization or other suitable optimization process to adjust parameters in the ML model 126 to enable the predicted residual error outputs of the ML model 126 to converge with the ground-truth residual error data. In the illustrative embodiment of FIG. 1, during the training process, the system 104 updates the parameter values of various elements in the ML model, such as the parameter values in one or more Gated Recurrent Units (GRU) of a GRU encoder/decode network, based on the observed differences between the output of the ML model 126 and the ground-truth residual error data. As is known in the art, the system 104 performs this training process iteratively over a large number of individual data series in the training analytical data 124 to enable the ML model 126 to generate useful residual error estimates for a wide range of potential inputs while avoiding over-fitting to the training data. This training process is considered a "supervised" training process because *a priori* labeled ground-truth residual error data are available to the training process, but other configurations utilize an unsupervised training process, which does not utilize the ground-truth data during training. In one configuration, the training process continues iteratively until the plurality of parameter values in the ML model 126 converge over at least two successive training operations. In another configuration, the training process continues iteratively until the plurality of predicted error values corresponding to each biomarker measurement sequence converge over at least two successive training operations. In yet another configuration, the training process performs the one or more training operations a predetermined number of times to generate the trained machine-learning model. Upon completion of the training process, the training system 104 stores the trained ML model 126 in the memory 120 for use in further inferencing operations, which are described in further detail below. In alternative configurations, the trained ML model 126 may be distributed to other computing systems via the network 150 or other data transfer mechanism to enable other computing systems to perform inferencing with the ML model 126, even if those systems did not directly participate in training the ML model 126.

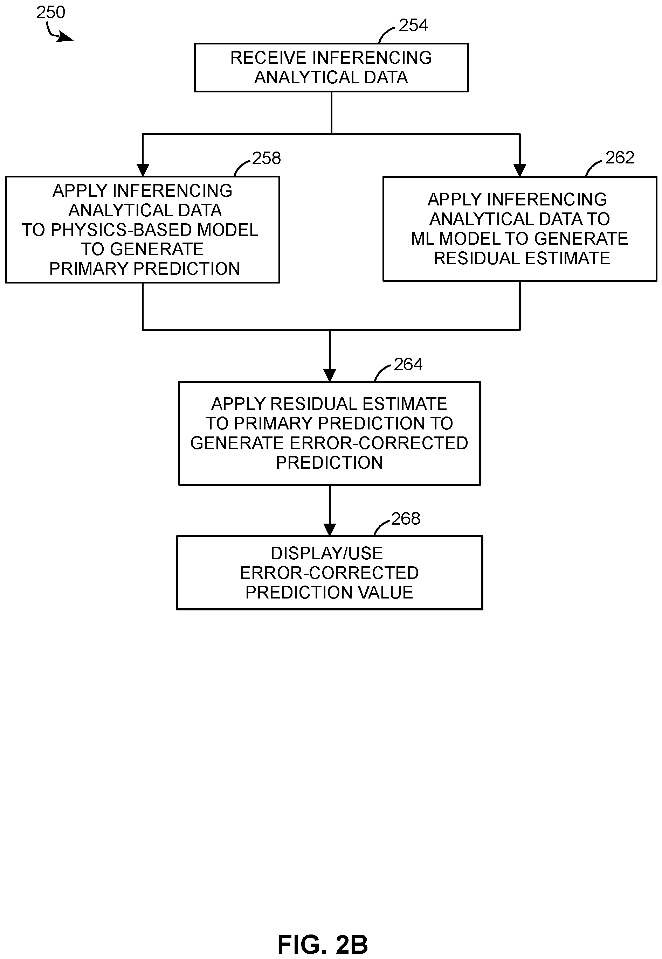

FIG. 2B is a block diagram of a process 250 for generating predictions of biomarkers using the ML model 126 and the physics-based model 140 in the system 104 of FIG. 1 after the ML model 126 is trained using the process 200 described above. In the description below, a reference to the process 250 performing a function or action refers to the operation of a processor to execute stored program instructions to perform the function or action in coordination with other components of a biomarker prediction and machine learning system. In the illustrative embodiment of FIG. 2B, the process 250 performs an inferencing operation with the trained ML model 126 to identify residual errors from a physics-based model that predicts future blood glucose levels in a PwD, but those of skill in the art will recognize the applicability of this process to physics-based prediction models for other biomarkers as well. The process 250 is described in conjunction with the embodiment of FIG. 1 and the system 104 acting as an inferencing system for illustrative purposes.

During the process 250, the inferencing system 104 receives inferencing analytical data from one of the inferencing sensor device systems 164 that collects the analytical data from one of the inferencing users 162 (block 254). For example, in a blood glucose prediction system a CGM sensor collects a series of glucose measurements that correspond to the blood glucose levels of an inferencing user 162 over time. The CGM transmits the blood glucose measurement data to the inferencing system 104 either directly via the network 150 or via an intermediate electronic device such as a smartphone, PC, or other similar electronic device in the inferencing sensor device system 164. The inferencing system 104 stores the inferencing analytical data 132 in the memory 120 for additional processing. In one alternative configuration, the inferencing sensor is a spot monitoring BGM instead of, or in addition to, a CGM. In still another alternative configuration, the CGM or BGM is supplemented by one or more additional sensors including cardiac monitors, respiratory monitors, pulse oximetry devices, physical activity monitors, and the like that generate additional inferencing analytical data. In general, while the hardware used for the training sensors 154 need not be identical to the hardware for the inferencing sensor device systems 164, preferentially the sensors in the inferencing sensor device systems 164 provide inferencing analytical data that is comparable in accuracy and precision to the training analytical data 154 to ensure that the physics-based model 140 and the trained ML model 126 in the inferencing system 104 can perform inferencing operations with consistent data to that employed during the training process. In the configuration of FIG. 1, the inferencing system 104 is a centralized system that performs inferencing for multiple inferencing users 162, but each individual inferencing operation only processes the inferencing analytical data to produce a biomarker prediction for a single one of the inferencing users 162.

Referring again to FIG. 2, the process 250 continues as the inferencing system applies the inferencing analytical data to the physics-based model to generate a primary biomarker prediction (block 258), and applies the inferencing analytical data to the ML model to generate a residual error estimate corresponding to the output of the physics based model (block 262). In the inferencing system 104, the processor 108 applies the inferencing analytical data 132 to the physics-based model 140 to generate the primary biomarker prediction, such as a sequence of one or more future predicted blood glucose values. The processor 108 also applies the inferencing analytical data 132 to the ML model 126 to generate the residual error estimate for the physics-based model 140, such as a sequence of one or more future residual error values corresponding to the primary biomarker predictions. The processing described with reference to blocks 258 and 262 in FIG. 2 may be performed in any order or concurrently.

The process 250 continues as the processor 108 applies the residual error estimate 136 to the primary prediction biomarker output data 144 to generate the corrected biomarker output data 148 (block 264). Using blood glucose as an exemplary biomarker, if the physics-based model 140 generates a predicted value, such as a prediction of 120 mg/dL, and the ML model 126 produces a residual error estimate of -5 mg/dL, then the processor 108 generates a corrected biomarker value of 115 mg/dL. In this embodiment, the processor 108 generates the corrected biomarker value via direct addition and subtraction of the estimated residual error with the predicted biomarker value, although in other configurations a different numeric operation may be applied to generate the error-corrected prediction value. As described above, in some embodiments the physics-based model 140 generates a series of future predicted biomarker values, such as a series of twenty-four (24) blood glucose values at five (5) minute increments for a two-hour blood glucose prediction, and the ML model 126 generates a corresponding series of residual error estimates for each prediction from the physics-based model to generate a series of corrected predicted blood glucose values. In other configurations, the physics based model and the ML model generate either a single prediction or a different number of predictions for the biomarker with associated predicted residual errors.

In the embodiment described above, the ML model 126 generates a single residual error estimate corresponding to the estimated error from the primary prediction of the physics-based model 140 for each future predicted blood glucose value. For many forms of ML model, the single prediction value is selected from multiple possible outputs from the ML model as the value having the highest probability estimate of being the correct residual error. However, in an alternative configuration the ML model generates the estimated error of each prediction from the physics-based model as a probability distribution that indicates a potential range of residual errors instead of identifying only a single residual error. In yet another configuration, the processor 108 generates the final residual error estimate as an average from multiple outputs of the ML model 126 where the individual outputs that form the average each exceed a predetermined minimum probability value. The inferencing system 104 then corrects the predicted biomarker form the physics-based model 140 based on multiple estimated errors from the ML model 126 having probability values exceeding a predetermined threshold in the probability distribution to generate the corrected prediction of the future value of the biomarker as a range of values based on the multiple error correction values.

Referring again to FIG. 2, the process 250 continues with the display or further use of the error-corrected prediction value (block 268). In one configuration, the inferencing system 104 transmits the corrected biomarker output data 148 to an electronic device that is associated with the inferencing sensor system 164 that collected the inferencing analytical sensor data. For example, a smartphone, smartwatch, tablet, PC, or other electronic computing device receives the corrected biomarker output data 148 via the network 150 and displays the biomarker data as a graph, numeric values, or other visual representation. In an alternative configuration in which the electronic device of an inferencing user 162 performs the biomarker prediction process using the physics-based model and the error estimation with the ML model 126 locally, the electronic device displays the corrected biomarker prediction value or values directly. In addition to, or instead of, a direct display to the inferencing user 162, the inferencing system 104 or any other computing system that performs the inferencing optionally transmits the corrected biomarker data to the HCP terminals 174 for display and additional analysis as part of a course of medical treatment. In addition to displaying the corrected biomarker prediction data, in other configurations the corrected biomarker output data 148 are stored in the memory 120 of the inferencing system 104 or in other medical systems, including electronic healthcare record (EHC) systems, for use in further diagnostic and treatment protocols.

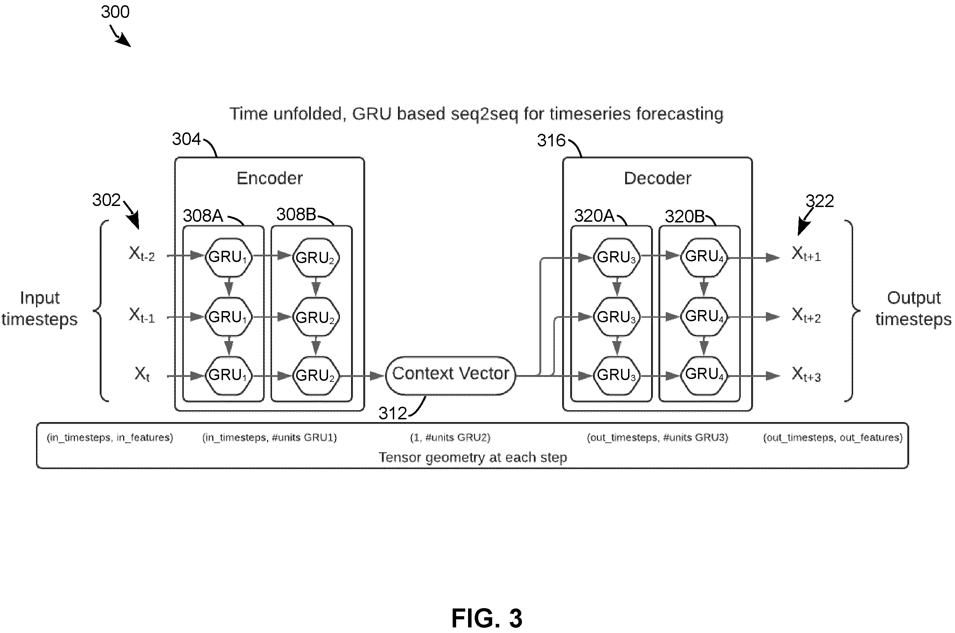

While numerous ML model types may be used for the ML model 126 in the system 104, one class of ML models that are suited to the prediction of sequences of residual outputs are recurrent neural networks (RNNs), and one more specific example of an RNN is a Sequence-to-Sequence (Seq2Seq) model that employs a Gated Recurrent Unit (GRU) encoder/decoder neural network. Other non-limiting examples include long-short term memory (LSTM) and transformer RNN models. FIG. 3 depicts an illustrative embodiment of a GRU encoder/decoder ML model 300 that is suitable for use with the training and inference processes to generate residual error estimates for a corresponding physics-based model in the system 104 as described herein. The ML model 300 includes an encoder 304 that is formed from two GRU layers 308A and 308B that each encodes data from a series of analytical data inputs. The GRUs in the encoder 304 generate a context vector 312, which serves as the initial context state for a second set of GRU layers 320A and 320B in a decoder 316. The decoder 316 generates an output corresponding to a series of predicted residual errors for corresponding series of biomarker predictions from the physics-based model 140. In the example of FIG. 3, the output GRU layer 320B in the decoder 316 generates a residual output error estimate for each time step in the output of the physics-based model 140. In the example of FIG. 3, the ML model 300 receives input as a one-dimensional input vector 302 of previously observed time series glucose measurement data in the example of FIG. 3, and generates an output as a one dimensional output vector 322 of a time series of estimated residual error values corresponding to predictions from the physics-based model 140.

Referring again to the encoder 304, as is known in the art, the first GRU layer 308A receives a time-series input data set 302 as a one-dimensional time-series input vector corresponding to the previously measured glucose values, which corresponds to the analytical input data at each step in the series. For example, in a time-series of glucose data, each input represents one measurement in one of the series of glucose measurements stored in the analytical training data, which are the same data that the physics-based model receives as inputs. The input to each GRU may also be a single or multi-dimensional vector that encodes glucose measurements and other data that are pertinent to the physics-based model including medication bolus data, carbohydrate consumption data, and physical activity data. In some configurations, the input vector data are normalized to numeric values within a predetermined range, such a range of 0.0 to 1.0 or another suitable input range.

While FIG. 3 depicts multiple GRUs in each layer for illustrative purposes, in at least one practical embodiment each layer only includes one GRU with parameters that are adjusted during the training process, and the input vector. For example, a single GRUi in layer 308A receives the time series data iteratively in the time series Xₜ₋₂, Xₜ₋₁, Xₜ and generates a series of context vector outputs that are based on the trained internal parameters of the GRU, the present input, and partially retained state data corresponding to prior inputs. In the example of FIG. 3, each GRU further incorporates two "gates" that serve to update the context vector data in each GRU. An "update" gate combines the present input to the gate with the existing context data for passing data to the next GRU in the encoder 304. A set of numeric weight values, which are generated during the training process, determines the portions of the prior context data to be passed to the next GRU. In the case of the first data input (X _{t-2_}in the example of FIG. 3) , for which there are no prior context data, the initial prior context vector is set to a zero vector or to another suitable initial vector value. A second "reset" gate uses a second set of numeric weights, which are generated during the training process, to determine a second combination of the input data and the context data that determines how much of the prior context data should be deleted or "forgotten" when passed to the next iterative operation of the GRU. Both gates typically apply a non-linear function (e.g. a sigmoid, hyperbolic tangent, rectified linear unit (ReLu), or similar) to the output to remove undesired linearity. The context vector output from each GRU corresponds to a combination of the outputs of the update and reset gates. While FIG. 3 depicts a simplified time series including three biomarker measurements for illustrative purposes, in one practical configuration the input biomarker measurement data include an input of twenty-four (24) inputs corresponding to glucose measurements generated at five minute intervals during a two hour monitoring window, although other .

The encoder 304 of FIG. 3 depicts two GRU layers 308A and 308B for illustrative purposes in a "deep" machine-learning model. The second GRU layer 308B includes a single GRU (GRU₂) that operates in a similar manner to the first GRUi in the layer 308A, but the GRU layer 308B receives the output of the GRU layer 308A in each iterative operation instead of receiving the original biomarker input data 302. The second GRU layer 308B enables the encoder 304 to extract additional latent information that may not be immediately identifiable from the original input data vector 302. The output context vector 312 from the encoder 304 is the final context vector generated the final iteration of the second GRU layer 308B, and encodes latent data from the original biomarkers that enable the decoder 316 to generate estimates of the residual error in the predictions of the physic-based model. While FIG. 3 depicts two GRU layers 308Aand 308B in the encoder 304, in an alternative configuration an encoder may also include a single GRU layer or three or more GRU layers as well. Furthermore, one or more GRUs may be used in each layer, and the number of GRUs need not be equal between layers of the encoder 304.

Referring again to FIG. 3, the context vector 312 is a context vector of the output from the final GRU layer in the encoder 304, which is the GRU layer 308B in the embodiment of FIG. 3. The context vector 312 stores the final encoded state generated by the GRU layers in the encoder 304, and the context vector 312 provides the initial input to the decoder 316.

In FIG. 3, the decoder 316 further comprises one or more GRU layers, which are depicted as two GRU layers 320A and 320B for illustrative purposes. The output of the decoder 316 represents the estimated error that the corresponding physics-based model is predicted to generate at each time point in a series of predictions. For example, in the embodiment of FIG. 3, the GRU layers 320A and 320B each include a single GRU (GRU₃ and GRU₄, respectively) that uses the same update and reset gate structure described above, but operate differently in that the decoder GRUs do not receive direct biomarker input data. Instead, the context vector 312 from the encoder 304 is provided as the initial context for the first GRU layer 320A in the decoder 316. The GRU layers in the decoder 316 then generate a sequence of outputs based only on the context data and the internal numerical weight values for the update and reset gates that are generated during the training process. In more detail, the decoder GRU layer 320A receives the context vector 312 as an input, and generates an output sequence that is provided to the GRU layer 320B, which generates a residual estimate output sequence 322. In the ML model 300, the decoder 316 generates an output that corresponds to a predicted residual error corresponding to the primary predicted biomarker value that the physics-based model 140 generates at the given time increment, such as a sequence of twenty-four (24) time increments at five (5) minute intervals for residual error estimates that correspond to a two-hour prediction period from the physics-based model 140.. In some configurations, the residual error estimate output sequence 322 is generated in a normalized numeric range, such as 0.0 to 1.0 or another numeric range, which is then scaled to match the expected numeric range of estimated residual error values for biomarker predictions. In the example of FIG. 3, the decoder 316 includes two GRU layers 320A and 320B for illustrative purposes, but other configurations may include a single GRU layer or three or more GRU layers.. Furthermore, one or more GRUs may be used in each layer, and the number of GRUs need not be equal between layers of the decoder 316. Additionally, the encoder 304 and decoder 316 may use different numbers of GRU layers and numbers of GRUs in each layer in alternative configurations. While the examples depicted herein describe a model for estimating residual errors that uses a sequence of input data of equal length to the sequence of output data (e.g. twenty-four input and output values corresponding to two hours of prior data and future estimated residual errors), in other configurations the input and output sequences may have different lengths. In particular, one alternative configuration generates only a single estimated residual value that represents an estimate of error in a present time biomarker prediction or a short-term future biomarker value prediction for the user in an interstitial time between direct measurements from the corresponding sensor device..

The GRU ML model 300 of FIG. 3 is one illustrative example of a suitable recursive neural network (RNN) machine learning model that is particularly suited to predicting a series of outputs, but the GRU ML model 300 is also a non-limiting example, and other ML models may be used as well. For example, the GRUs described above are known to the art as "fully gated" GRUs since they include both update and reset gates, but other forms of GRUs such as minimal and light gated recurrent units may be used as well. Still more broadly, GRUs are but one sub-category of a broader range of RNNs including long-short term memory (LSTM) and transformer based RNNs, both of which may be used in alternative embodiments. Even more broadly, while RNNs are especially suited to situations in which an ML model generates a sequence of residual error estimates, in other configurations in which the ML model is only configured to generate a single residual error estimate corresponding to a single output of a physics-based model, then a different form of ML model including, but not limited to, a convolutional neural network, random forest classifier, perceptron, support vector machine, Bayesian network, and the like may be used instead.

In the embodiment described above, after training the ML model 126 generates an output sequence of predicted residual values based on the input of inferencing analytical data 132 from a measurement device, such as a CGM or other suitable blood glucose measurement device, for a prior time range, which is also generally the same data that the physics-based model 140 uses to generate the primary biomarker prediction. However, in other configurations the ML model 126 performs training and inferencing using somewhat different sets of input data. For example in one alternative configuration, the ML model 126 receives both the prior time analytical data and prior time calculations of the actual residual error between earlier predictions and the observed biomarker data at each time point during training and inferencing. In still another configuration, the ML model 126 receives the primary biomarker prediction values from the physics-based model 140 in addition to, or instead of, either or both of the analytical data and the prior time residual error calculations during training and inferencing.

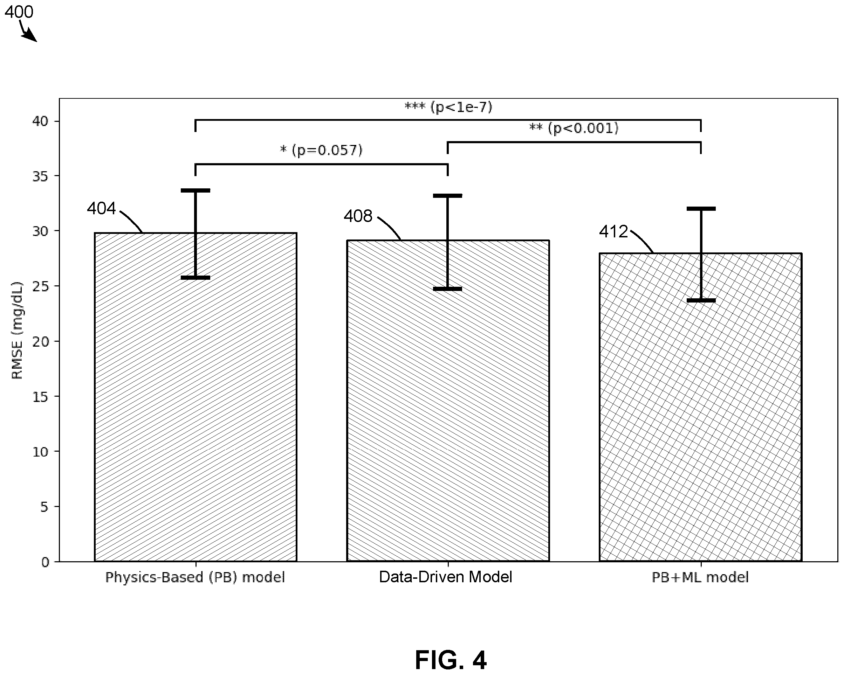

FIG. 4 depicts an example graph 400 of root mean squared error (RMSE) results of errors in biomarker predictions generated by a physics-based prediction model (PB model 404), a data-driven ML model (data-driven model 408), and the physics-based model with additional error correction from the ML model (PB+MI, 412) that is described above. The graph depicts the RMSE and standard deviations in units of milligrams/deciliter of glucose in blood. In more detail, the error results 404 depict the RMSE for the physics-based model 140 in the system 104 without the additional correction provided by the ML model 126. The data-driven model RMSE graph 408 represented by the error bar is a trained "seq2seq" prior-art recurrent neural network (RNN) that employs a first set of forty-eight (48) input buckets to encode a four hour time period of glucose values prior to the prediction and a second set of twenty-four (24) buckets to generate a prediction sequence of glucose biomarker values. The error data in the error bar 408 are determined empirically by comparing the predictions of the data-driven model to observed glucose measurements. Once again, while the structure of this seq2seq data-driven model has similarities to that of the ML model 126 in the system 104, this data-driven biomarker prediction model generates estimated predictions of the biomarker, such as glucose, directly instead of generating estimates of errors corresponding to the outputs from a physics-based model, as is described above. As such, this seq2seq model does not form part of the system 104, and is described here as an example of a state-of-the-art purely data-driven ML prediction model. The RMSE graph 412 depicts the final output from the system 104 that includes the primary prediction from the physics-based model 140 and the applied correction based on the error estimate from the ML model 126. As depicted in FIG. 4, the PB+MI, results 412 shows an improvement with reduced RMSE relative to the original physics-based model 404 and reduced RMSE compared to the state-of-the-art data-driven model 408 RMSE results. Represented numerically, the physics-based model results 404 produced an RMSE of 29.72±4.01 mg/dL, the data-driven model 408 results produced an RMSE of 29.00±4.33 mg/dL, and the combined physics-based model with the ML residual estimation model results 412 produced an RMSE of 27.87±4.16 mg/dL. In particular, the system 104 generates results with the lowest RMSE out of the three, and with a standard deviation of RMSE that lies between the physics-based model 140 and the data-driven seq2seq model. The graph 400 further includes statistical significance "p" values indicating the probability of the observed results being randomly generated. In particular, the probability that the observed RMSE from the physics-based model 140 (404) and the complete PB+MI, results (412) are generated randomly is p < 10⁻⁷ in the example of FIG. 4, which is generally accepted as being statistically significant by those of skill in the art.

This disclosure is described in connection with what are considered to be the most practical and preferred embodiments. However, these embodiments are presented by way of illustration of the improvements to the art described herein, and these improvements are not strictly limited to the disclosed embodiments. Accordingly, one of skill in the art will realize that this disclosure encompasses all modifications and alternative arrangements within the spirit and scope of the disclosure and as set forth in the following claims.

## Claims

1. A method for biomarker prediction comprising:
providing a set of program instructions which, when stored in a non-transitory memory and executed by a processor in a computing system, configure the computing system to:
receive data corresponding to at least one measurement of a biomarker value in a body of a user;
generate a first prediction of a future value of the biomarker in the body of the user based on the at least one measurement applied to a physics-based model;
generate an estimated error of the first prediction based on the at least one measurement using a trained machine learning model;
correct the first prediction based on the estimated error to generate a second prediction of the future value of the biomarker in the body of the user; and
generate an output indicating the second prediction of the future value of the biomarker in the body of the user.

2. The method of claim 1, wherein the biomarker is glucose.

3. The method according to any one of the preceding claims, wherein the physics-based model is a Patek model, wherein the physics-based model is a Patek model that incorporates a Kalman filter.

4. The method according to any one of the preceding claims, wherein the physics-based model is a Bergman model.

5. The method according to any one of the preceding claims, wherein the at least one measurement of the biomarker value in the body of the user further comprises a time series of biomarker measurements received from a continuous biomarker measurement device coupled to the body of the user.

6. The method of claim 5, wherein the trained machine learning model is a recurrent neural network (RNN), wherein the RNN further comprises an encoder, and a decoder.

7. The method according to any one of the preceding claims, wherein the program instructions further configure the computing system to:
generate the estimated error of the first prediction as a probability distribution corresponding to an output of the trained machine learning model; and
correct the first prediction based on a plurality of estimated errors having probability values exceeding a predetermined threshold in the probability distribution to generate the second prediction of the future value of the biomarker as a range of values.

8. A method for training a machine learning model for use in predicting biomarker value comprising:
receiving, with a training system, a training data set, the training data set further comprising a plurality of biomarker measurement sequences;
generating, with the training system, at least one predicted biomarker value for each biomarker measurement sequence based on a first portion of each biomarker measurement sequence using a physics-based model;
identifying, with the training system, a ground-truth error in the at least one predicted biomarker value for each biomarker measurement sequence based on a second portion of each biomarker measurement sequence; and
performing, with the training system, one or more training operations in an iterative manner to generate a trained machine learning model from the untrained machine learning model, each training operation comprising:
applying, with the training system, the first portion of each biomarker measurement sequence to an untrained machine learning model to generate a plurality of predicted error values corresponding to each biomarker measurement sequence; and
adjusting a plurality of parameter values in the untrained machine learning model based on a difference between the predicted error value and the ground-truth error value corresponding to each biomarker measurement sequence to optimize the output of the untrained machine learning model.

9. The method of claim 8, wherein the training system performs a gradient descent optimization to adjust the plurality of parameter values in the untrained machine learning model during each training operation.

10. The method according to any one of the preceding claims relating to a method for training a machine learning model further comprising:
performing the one or more training operations a predetermined number of times to generate the trained machine learning model, and/or
performing the one or more training operations iteratively until the plurality of parameter values converge over at least two successive training operations, and/or
performing the one or more training operations iteratively until the plurality of predicted error values corresponding to each biomarker measurement sequence converge over at least two successive training operations.

11. The method according to any one of the preceding claims relating to a method for training a machine learning model, wherein the biomarker is glucose.

12. The method according to any one of the preceding claims relating to a method for training a machine learning model, wherein the training data set further comprises a plurality of time series of biomarker measurements received from a plurality of continuous biomarker measurement devices, each continuous biomarker measurement device being coupled to a body of a training user in a plurality of training users.

13. The method according to any one of the preceding claims relating to a method for training a machine learning model, wherein the untrained machine learning model is an untrained recurrent neural network (RNN) and the trained machine learning model is a trained RNN.

14. The method according to any one of the preceding claims relating to a method for training a machine learning model, wherein the physics-based model is a Patek model, wherein the physics-based model is a Patek model that incorporates a Kalman filter.

15. The method according to any one of the preceding claims relating to a method for training a machine learning model, wherein the physics-based model is a Bergman model.

16. A system for biomarker prediction comprising:
a memory configured to store program instructions, a physics-based model, a trained machine learning model, and data corresponding to at least one measurement of a biomarker value in a body of a user;
an output device; and
a processor operatively coupled to the memory and the output device, the processor being configured to:
execute the stored program instructions to perform any of the methods of claims 1 - 7 to generate an output of the second prediction of the future value of the biomarker in the body of the user using the physics-based model and the trained machine learning model with the output device.

17. A system for training a machine learning model for use in predicting biomarker value comprising:
a memory configured to store program instructions, a physics-based model, an untrained machine learning model, and a training data set; and
a processor operatively coupled to the memory, the processor being further configured to:
execute the stored program instructions to perform any of the methods of claims 8 - 15 to generate a trained machine learning model using the physics-based model, the untrained machine learning model, and the training data set.
